(19) Europäisches Patentamt
European Patent Office
Office européen des brevets

(11) **EP 3 419 503 B1**

(12) **EUROPÄISCHE PATENTSCHRIFT**

(45) Veröffentlichungstag und Bekanntmachung des Hinweises auf die Patenterteilung:
**03.04.2024 Patentblatt 2024/14**

(21) Anmeldenummer: **17708193.2**

(22) Anmeldetag: **22.02.2017**

(51) Internationale Patentklassifikation (IPC):
***A61B 5/00*** *(2006.01)*

(52) Gemeinsame Patentklassifikation (CPC):
**A61B 5/7203; A61B 5/24;** A61B 5/6846

(86) Internationale Anmeldenummer:
**PCT/EP2017/054057**

(87) Internationale Veröffentlichungsnummer:
**WO 2017/144529 (31.08.2017 Gazette 2017/35)**

(54) **DIGITALES BIOPOTENTIALERFASSUNGSSYSTEM MIT 8 KANÄLEN**

DIGITAL BIOPOTENTIAL ACQUISITION SYSTEM HAVING 8 CHANNELS

SYSTÈME DE DÉTECTION DE BIOPOTENTIEL NUMÉRIQUE À 8 CANAUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.02.2016 DE 102016103073**

(43) Veröffentlichungstag der Anmeldung:
**02.01.2019 Patentblatt 2019/01**

(73) Patentinhaber: **Neuroloop GmbH**
**79108 Freiburg (DE)**

(72) Erfinder:
• KUHL, Matthias
  79108 Freiburg (DE)
• MANOLI, Yiannos
  79104 Freiburg (DE)
• PLACHTA, Dennis
  79279 Vörstetten (DE)
• STIEGLITZ, Thomas
  79110 Freiburg (DE)
• COTA, Oscar
  79114 Freiburg (DE)

(74) Vertreter: **Winter, Brandl - Partnerschaft mbB**
**Alois-Steinecker-Straße 22**
**85354 Freising (DE)**

(56) Entgegenhaltungen:
US-A1- 2003 097 050      US-A1- 2005 116 773
US-A1- 2010 106 041      US-A1- 2014 180 052
US-A1- 2014 330 102      US-B1- 7 385 443
US-B1- 8 436 679

• SITI NUR SYUHADAH BAHARUDIN ET AL: "Design and analysis of a two-stage OTA for sensor interface circuit", 2014 IEEE SYMPOSIUM ON COMPUTER APPLICATIONS AND INDUSTRIAL ELECTRONICS (ISCAIE), 30. April 2014 (2014-04-30), Seiten 1-5, XP055366201, DOI: 10.1109/ISCAIE.2014.7010215 ISBN: 978-1-4799-4352-4

**EP 3 419 503 B1**

## Beschreibung

Die Erfindung betrifft ein biokompatibles, neuronales Implantat zur Aufzeichnung (Recording) von neuronalen Signalen in einem Lebewesen. Insbesondere offenbart die vorliegende Erfindung ein innerhalb eines Chips gelegenes Erfassungssystem für neurone Signale zur Implantation in ein Lebewesen.

## Stand der Technik

Unter Biopotential wird ein elektrisches Potential verstanden, das zwischen Punkten in lebenden Zellen, Gewebe und Organismen gemessen wird und das im Zusammenhang mit allen biochemischen Prozessen auftritt. Es beschreibt zudem den Transfer von Informationen zwischen und innerhalb von Zellen. Es ist eine elektrische Größe (Spannung, Strom oder Feldstärke), die von chemischen Reaktionen geladener Ionen hervorgerufen wird. Der Begriff findet weitere Verwendung in der Beschreibung des Transfers von Informationen zwischen und innerhalb von Zellen, beispielsweise bei der Signalübertragung.

Neuronale Implantate können Signale von einzelnen Neuronen oder Gruppen von Neuronen (biologische neuronale Netzwerke) in einem Lebewesen elektrisch stimulieren, erfassen und blockieren (oder auch gleichzeitig erfassen und stimulieren).

Die vorliegende Erfindung offenbart die Ausgestaltung und den Test eines integrierten CMOS-Biopotentialerfassungschips mit 8 Kanälen, der aus einem rauscharmen Verstärker (engl.: low-noise amplifier, LNA), einer zweiten Stufe, einem Multiplexer und zwei Analog-Digital-Wandlern (engl.: analogue-to-digital converter, ADC) besteht.

Aufgrund seines variablen Leistungsverbrauchs kann das integrierte Rauschen der ersten Stufe von 1,94 auf 0,693 $\mu V_{RMS}$ ($I_{ss}$ = 250 $\mu A$) herabjustiert werden. Die Vorrichtung hat variable untere und obere Eckfrequenzen und gibt zwei digitale 16-Bit-Datenströme bei 1 Mb/s aus.

Das Chip Die ist in X-Fab-0,35-$\mu$m-CMOS-Technologie hergestellt und hat eine Fläche von 10 mm$^2$.

Neuronale Implantate sind Vorrichtungen, welche die Behandlung von Krankheiten wie beispielsweise Parkinson, Hörbeeinträchtigungen und Herzfehlern unterstützen.

Derartige Vorrichtungen verbinden das Nervensystem durch elektrische Stimulation, um eine Reaktion des Körpers hervorzurufen. Cochlear-Implantate stimulieren zum Beispiel den Hörnerv, um den Sinneseindruck des Hören zu erzeugen, Schrittmacher stimulieren die Innenwand des Herzen um Herzmuskelkontraktionen auszulösen und Tiefenhirnstimulatoren generieren Signale, die ungewollte, durch Parkinson ausgelöste Muskelzuckungen verhindern.

Die medizinische Forschung hat das Ziel, zu verstehen wie sich neuronale Implantate auf das Nervensystem auswirken sollten. Normalerweise werden bei Versuchen an Menschen und Tieren große Aufnahmesysteme verwendet, die es ermöglichen, Signale vom Gehirn oder Nerven sichtbar zu machen und zu verarbeiten. Aktuelle Versuche zeigen eine klare Tendenz hin zur Verwendung von implantierbaren Erfassungssystemen, da diese der Realität von medizinischen Implantaten einen Schritt näher kommen.

Je nach Art der Anwendung decken bioelektrische Signale eine große Bandbreite an Amplituden, Rauschpegeln und Frequenzbändern ab. Aus diesem Grund ist ein Aufnahmesystem, das seine Eigenschaften in Bezug auf die jeweiligen Anwendungen anpassen kann, äußerst erstrebenswert.

Ghovanloo [6] zeigt ein System mit extrem niedrigem Stromverbrauch, das Gehirnsignale erfassen kann und das eine variable Bandbreite und Funkübertragung umfasst. Harrison et al zeigt einen vielseitig einsetzbaren Erfassungsverstärker, der sich in Fall von Gehirnaktionspotentialen, Elektroenzephalografie (EEG), Elektrokardiografie (EKG) und Elektromyografie (EMG) bewährt hat.

Ein Nachteil dieser Erfassungssysteme ist ihr Rauschpegel von jeweils 4 $\mu V_{RMS}$ und 2 $\mu V_{RMS}$, der bei Anwendungen mit EEG- und Elektroneurographie (ENG) relativ hoch ist.

Verstärker generieren ebenfalls Rauschen, das in thermisches und Funkelrauschen unterteilt ist.

Die thermische Rauschdichte ist in Bezug auf die Frequenz konstant und ist proportional zum äquivalenten Widerstand des Transistors.

Die Funkelrauschdichte hingegen ist abhängig von der Frequenz mit einem Faktor 1/f und ist umgekehrt proportional zur Transistorfläche.

Man hat bereits einiges an Aufwand betrieben, um die Rauschbegrenzung zu überwinden. Eine nennenswerte Arbeit ist der BJT-Eingangs-Transkonduktanz-Operationsverstärker (VC-OP) (engl.: BJT-input operational transconductance amplifier (OTA)) für ENG, vorgeschlagen von R. Rieger und N. Donaldson.

Da BJT-Transistoren kein Funkelrauschen erzeugen, ist das resultierende eingangsbezogene Rauschen von 300 $nV_{RMS}$ deutlich geringer als das früherer Verstärker. Jedoch hat diese Architektur zwei schwerwiegende Nachteile:

1. Sie hat einen Restgleichstrom von 20 nA aus der Elektroden-Gewebe-Grenzfläche, der auf Dauer zur Korrosion an den Kontakten führen kann und

2. die Technologie ist "open loop", was dazu führt, dass die Verstärkung eine zufällige Variable ist, was für die "True-Tripole-Anordnung, die für eine Cuff-Elektroden Aufnahme verwendet wird, eventuell ein Problem darstellen kann.

Zudem wurde eine Chopper-Struktur vorgeschlagen, die das Signal auf eine Frequenz verschiebt, bei der das Funkelrauschen vernachlässigbar ist. Daraufhin wird das Signal ohne Funkelrauschen demoduliert. Leider benötigt der Chopper-Verstärker mindestens zehnmal mehr Bandbreite um sicherzustellen, dass das Signal ausreichend weit entfernt ist.

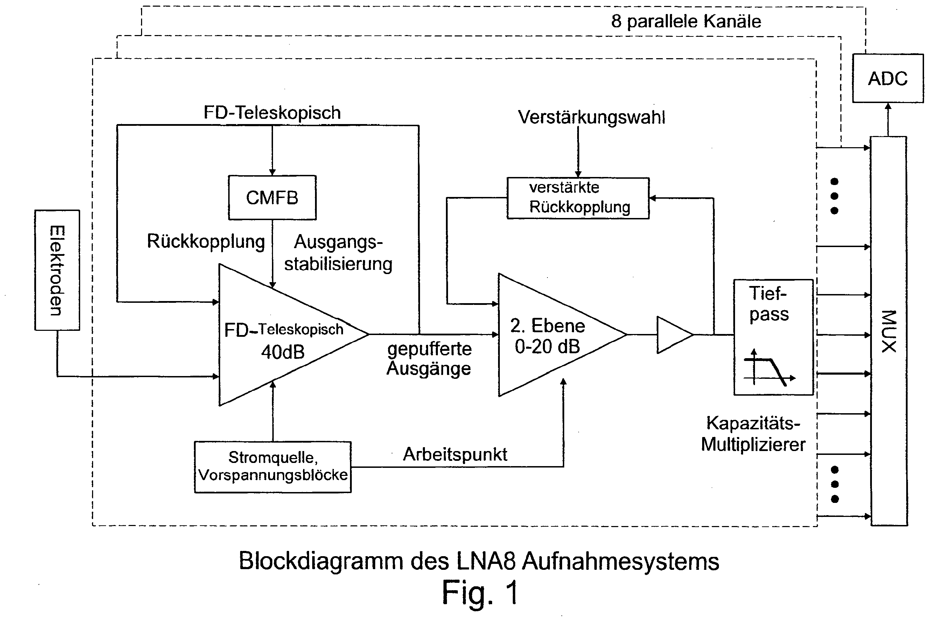

Diese Voraussetzung erhöht den Stromverbrauch des Verstärkers. In der vorliegenden Erfindung ist ein vielseitig einsetzbarer, rauscharmer Verstärker gezeigt, um einen Eingangsrauschpegel von sub-$\mu V_{RMS}$ zu erreichen. Der angewendete Ansatz zur Rauschminderung besteht aus angemessener Transistorgröße und -leistung und Verwendung von PMOS-Eingangstransistoren, die eine niedrigere Funkelrauschkonstante besitzen. Das vorliegende, in Figur 1 gezeigte System hat 8 bipolare Eingangskanäle und zwei unabhängige serielle Digitalausgänge mit zwei 10-Bit-ADCs. Das Dokument US 2003/097050 A1 offenbart ein bekanntes Bipotentialerfassungssystem.

## Erfindung

Die Erfindung ist wie in den Ansprüchen definiert.

## LNA-Vorverstärker

Es ist bekannt, dass die erste Stufe (Vorverstärker) in einer Verstärkerkette die wichtigste Stufe ist, da sie der für Rauschbelastung anfälligste Bestandteil ist. Aus diesem Grund wurde eine vollständig differentielle Teleskoparchitektur (Fully-differential Telesopic architecture) eingesetzt.

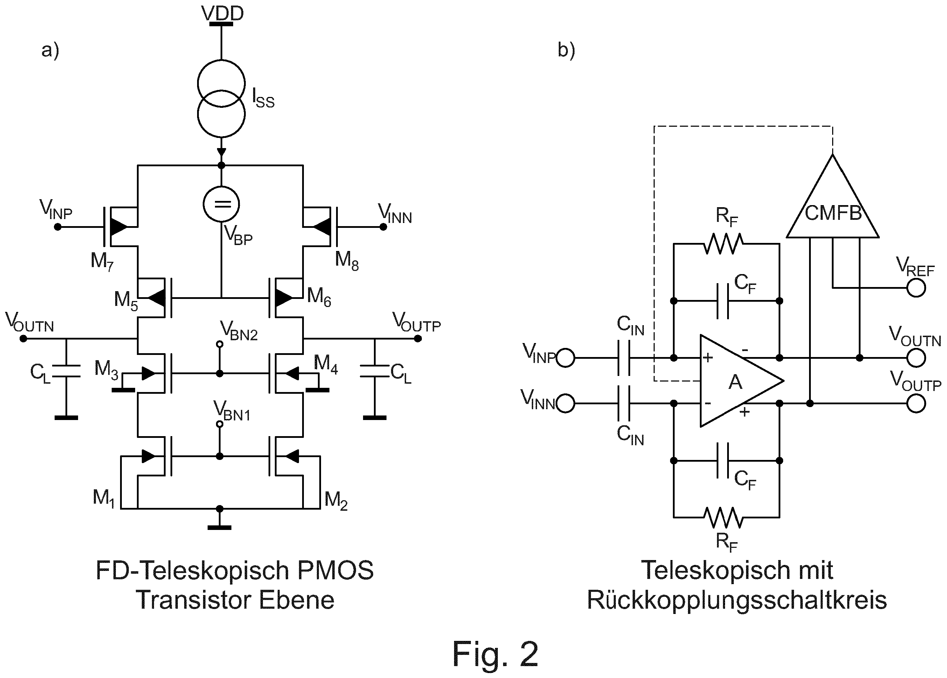

Die in Figur 2 gezeigte Architektur liefert hohe Verstärkung und Bandbreite in einer einzigen Stufe und theoretisch ein unendliches Gleichtaktunterdrückungsverhältnis (CMRR) und unendliche Störspannungsunterdrückung (PSRR).

Die Gleichungen der Verstärkerkanäle sind bekannt, umformuliert für die $g_m/I_D$-Ausgestaltungsmethodik, das Rauschmodel ist:

$$\overline{V_{n,in}^2} = \frac{16kT(\Delta f - 1)}{3\left(\frac{g_m}{I_D}\right)_{1,2}}\left(1 + \frac{\left(\frac{g_m}{I_D}\right)_{7,8}}{\left(\frac{g_m}{I_D}\right)_{1,2}}\right)\frac{1}{I_D} + \frac{2ln\Delta f}{C_{OX}}\left(\frac{K_n}{(WL)_{1,2}} + \frac{K_p}{(WL)_{7,8}}\left[\frac{\left(\frac{g_m}{I_D}\right)_{7,8}}{\left(\frac{g_m}{I_D}\right)_{1,2}}\right]^2\right) \quad (1)$$

Und die Übertragungsfunktion:

$$H(s) = 2\frac{C_{IN}}{C_F}\frac{\frac{s}{2\pi f_{cL}}}{(1 + \frac{s}{2\pi f_{cL}})(1 + \frac{s}{2\pi f_{cL}})} \quad (2)$$

Mit

$$f_{cL} = \frac{1}{2\pi R_F C_F}; f_{cU} = \frac{C_F}{C_{IN}}\frac{g_{m1,2}}{\pi C_L} = \frac{C_F}{C_{IN}}\frac{\sqrt{\beta_{1,2}}}{\pi C_L}\sqrt{I_{SS}} \quad (3)$$

## Legende:

| Kn | Funkelrauschkonstante | NMOS | $120 \times 10^{-24}$ V$^2$F |
|----|----------------------|------|------------------------------|
| Kp | Funkelrauschkonstante | PMOS | $20 \times 10^{-24}$ V$^2$F |

(fortgesetzt)

| | | |
|---|---|---|
| $k$ | Boltzmann-Konstante | $1{,}3806 \times 10^{-23} m^2 kg/s^2 K$ |

(1)

| | | |
|---|---|---|
| $V^2_{n,in}$ | Gesamtes Eingangsrauschen | $V_{RMS}$ |
| $k$ | Boltzmann-Konstante | 1 |
| $T$ | Temperatur | K |
| $gm$ | Transkonduktanz | A/V |
| $ID$ | Stromstärke am Drain-Anschluss | A |

(2)

| | | |
|---|---|---|
| $C_{IN}$ | Eingangskapazität | F |
| $C_F$ | Rückführungskapazität | F |
| $f_{cL}$ | untere Eckfrequenz | Hz |
| $f_{cU}$ | obere Eckfrequenz | Hz |
| $R_F$ | Rückführungswiderstand | $\Omega$ |
| $\beta$ | MOSFET-Transistor-Stromverstärkung | $A/V^2$ |
| $I_{SS}$ | Polarisierungsstrom für FD-Teleskopverstärker | A |

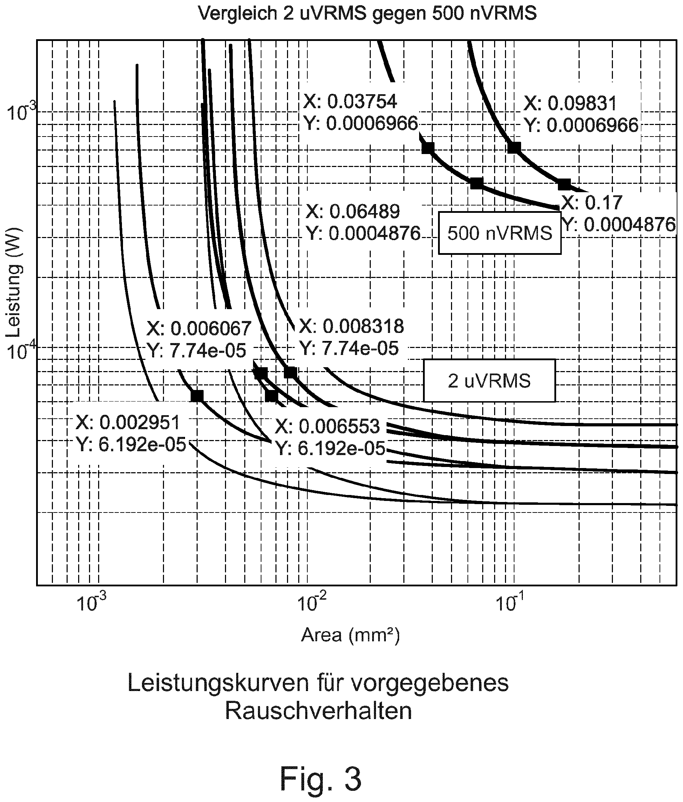

Unter Verwendung der PMOS-Transistoren und des optimalen Punktes, der in Figur 3 markiert ist, wurden die in folgender Tabelle gezeigten Größen ermittelt:

Größen des PMOS-FD-Teleskopverstärkers

| FD-teleskopisch | Parameter | |
|---|---|---|
| **Größen** | $W$ [$\mu$m] | $L$ [$\mu$m] |
| $M_{1,2}$ | 8000 | 1,5 |
| $M_{3,4}$ | 1728 | 0,5 |
| $M_{5,6}$ | 168 | 1 |
| $M_{7,8}$ | 288 | 24 |
| Belastungskapazität | 30,5 pF | |
| Biasstrom $I_{SS}$ | 205 $\mu$A | |
| Wirkfläche | 0,04 mm$^2$ | |
| Layoutfläche | 0,15 mm$^2$ | |
| Kanalfläche | $2028 \times 720$ $\mu$m$^2$ | |

Zweite Stufe

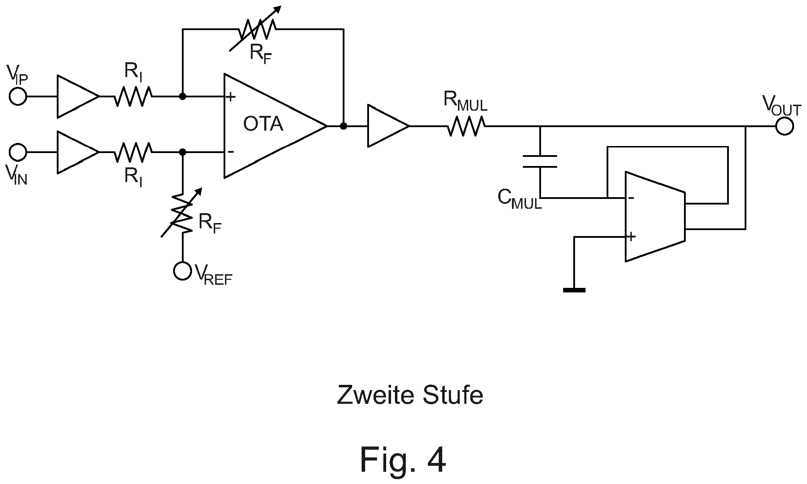

Die in Figur 4 gezeigte zweite Stufe ist für die FD (Fully-Differential) -zu-single-ended-Umwandlung verantwortlich mit einem Eingangsrauschen von 6 $\mu V_{RMS}$ für einen Leistungsverbrauch von 148 $\mu$W (11 $\mu V_{RMS}$ und 46 $\mu$W im LP-Modus). Durch die Rückkoppelung liefert sie eine Verstärkung von entweder 0 dB oder 20 dB. Der OTA besteht aus einem single-ended 2-Stufen-Miller-Verstärker.

Im Bereich der Elektronik bezeichnet der **Millereffekt** die Vergrößerung der äquivalenten Eingangskapazität eines invertierenden Spannungsverstärkers auf Grund der Verstärkung des Effekts der Kapazität zwischen den Eingangs- und Ausgangsanschlüssen. Die scheinbar erhöhte Eingangskapazität auf Grund des Millereffekts ergibt sich wie folgt:

$$C_M = C(1 + A_v)$$

wobei -$A_v$ die Verstärkung und C die Rückkopplungskapazität ist.

Auch wenn der Begriff *Millereffekt* sich normalerweise auf Kapazitäten bezieht, kann jede Impedanz, die zwischen dem Eingang und einem anderen Knoten, der eine Verstärkung zeigt, verbunden ist, die Verstärkereingangsimpedanz mit diesem Effekt modifizieren.

### Tiefpassfilter mit Kapazitätsmultiplizierer

Da unterschiedliche Anwendungen unterschiedliche obere Eckfrequenzen fcu benötigen, wurde ein variabler RC-Tiefpassfilter integriert.

In [3] wurde diese Variation durch Justierung des LNA-Vorstroms $I_{ss}$ erreicht, wodurch eine Variation des Rauschverhaltens erzeugt wurde. Um diese ungewollte Kopplung zu vermeiden, wurde ein Kapazitätsmultiplizierer vorgeschlagen, der den Steuerstrom-OTA aus [4] verwendet und der, wie in [5] beschrieben, mit der zweiten Stufe verbunden ist. Der Kapazitätsmultiplikationsfaktor (von 50 pF bis 5 nF) wird durch den Differentialeingang $V_{GC\pm}$, den Vorstrom von 56 µA und eine 0,013 mm$^2$ große Fläche eingestellt.

### MUX, Analog-Digital-Wandler und serieller Ausgang

Der Chip verwendet die X-Fab 0,35-µm-Bibliothek 10-Bit SAR-ADC und integriert einen benutzerdefinierten, Flip-Flop-basierten Parallel-Seriell-Wandler. Der Little-Endian-16-Bit-Ausgang wird wie in [4] zusammengefasst, wobei S die Start-Token-Bits (H L) sind, Bits C3-C0 stellen die Kanalnummer dar und Bits D9-D0 sind die ADC-Abtastwerte.

### Leistungsverbrauch

Der Leistungsverbrauch des Chips wird in folgender Tabelle zusammengefasst:

| (*sim values) | 1 St [mW] | 2 St + Vorspan. | Cap.mult. (mW) | ADCs | Total (sim) mW |
|---|---|---|---|---|---|
| 29 µA | 0,765 | 2,03 | 1,19 (optional) | 0,495 | 4,49 |
| 29 µA LP | | 1,23 | | | 3,68 |
| 210 µA | 5,54 mW | 2,03 | 1,19 (optional) | | 9,26 |
| 210 µA LP | | 1,23 | | | 8,46 |
| 271 µA | 7,15 mW | 2,03 | 1,19 (optional) | | 10,87 |
| 271 µA LP | | 1,23 | | | 10,06 |

### Ergebnis

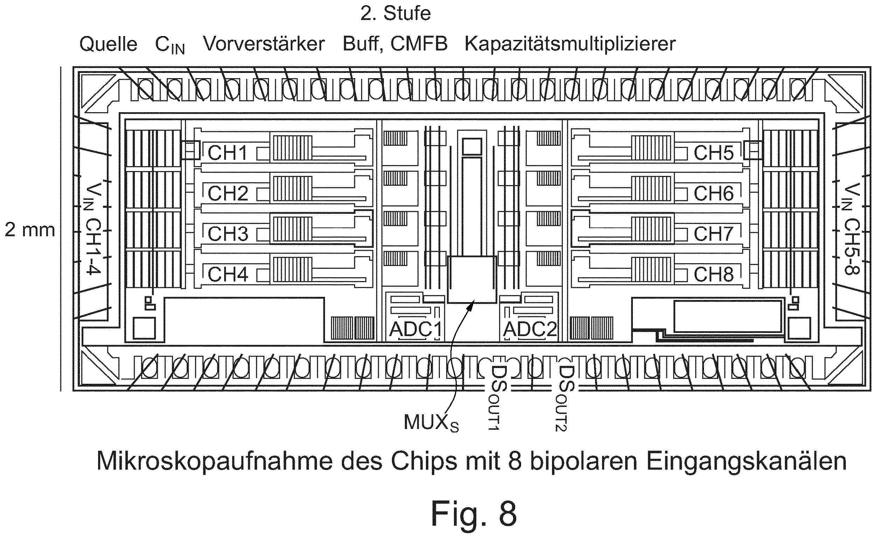

Der in Figur 8 gezeigte Chip wird mit X-Fab-0,35-µm-Technologie hergestellt. Die Verstärker-I/O-Pins werden in Tabelle 2 zusammengefasst. Die Systempins bieten Flexibilität für die Parameter:

- Funktion freigeben: stetige *ISS*-Variation, Zweite-Stufe Low-Power(LP)-Modus, Kapazitätsmultiplizierer, 20 dB Verstärkung

- Vorspannung: $V_{REF\_ISS}$, $V_{REF\_CMFB}$

- Frequenzbereichsvariation: $V_{GCP/N}$, $V_{TUNE}$

Obwohl der Chip für digitale Ausgabe gestaltet wurde, enthält er Test-Pins um seine Charakterisierung zu unterstützen, wie zum Beispiel die analogen Ausgänge des Vorverstärkers und des Tiefpassfilters von Kanal 1 und 5.

Tabelle 2, I/O-Pins des LNA8-Chips. Unterstrichene Pins sind Ausgänge.

| Analog | | Digital | | Test (analog) | |
|---|---|---|---|---|---|
| $(D,A)V_{DD}$ | 3 | $I_{SS}$(T1-T4) | 4 | $V_{EXTCMFB}$ | |
| $(D,A)V_{SS}$ | 3 | V_REF_ISS_EN | | $V_{DDISS(1,5)}$ | 2 |
| $V_{REF\ ISS}$ | | LP EN | | $\underline{V}_{OUTN/P(1,5)}$ | 2 |
| VIN(N/P)(1-8) | 16 | RESET EN | | $\underline{V}_{OUT1/5}$ | 2 |
| $V_{REF\ CMFB}$ | | GAIN_0dB_EN | | **Test (digital)** | |
| $V_{GCP/N}$ | 2 | CAP MULT EN | | RESET ADC | |
| $V_{TUNE}$ | | CLK | | $\underline{Q1}_{0-3}$ | 4 |
| $V_{REFH}$ | | $CHSEL_{0-3}$ | 4 | $\underline{EOC1}$ | |
| $V_{REFL}$ | | $\underline{DSOUT(1/2)}$ | 2 | SW1 | |

Die ADCs können mit zwei seriellen digitalen Ausgängen bis zu 1 MHz getaktet werden.

**Frequenzantwort**

Das LNA8-AUfnahmesystem weist jeweils durch Variation der Potentiale $V_{TUNE}$ und $V_{GC\pm}$ variable Eckfrequenzen fcU, fcL auf.

**Rauschverhalten**

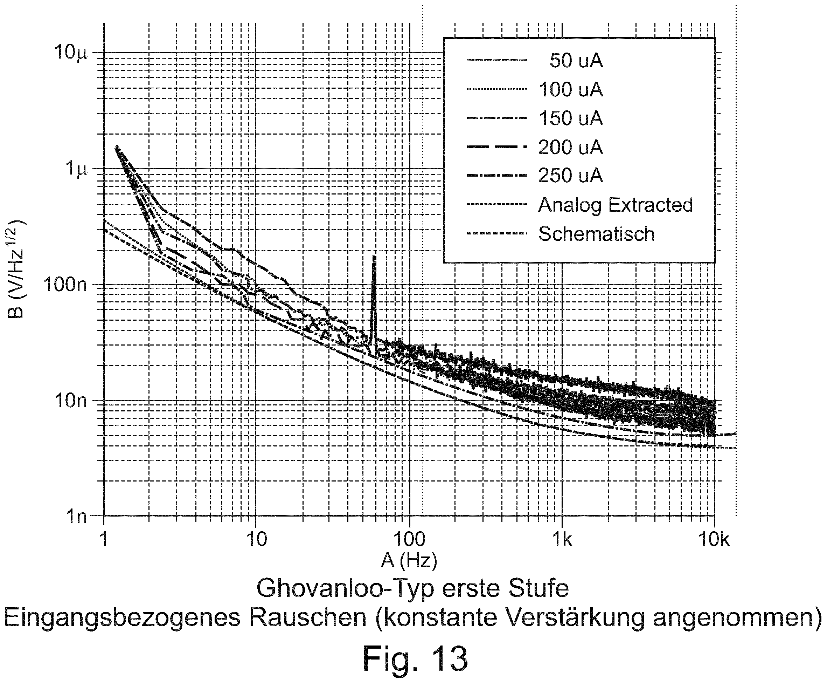

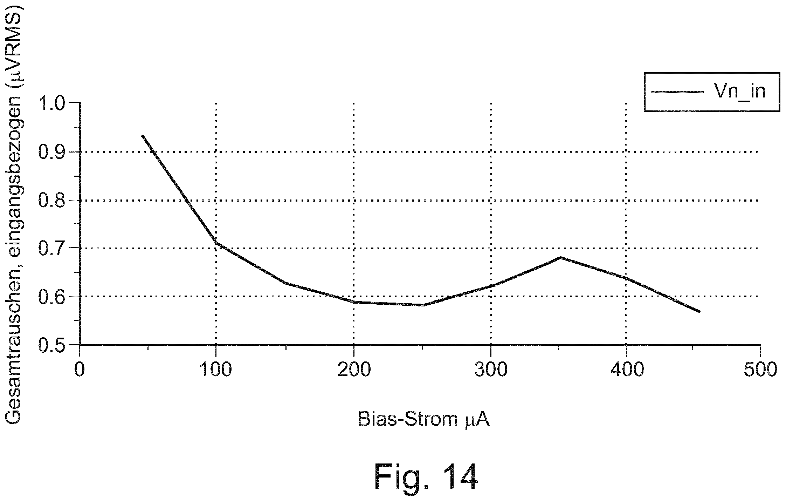

Die spektrale Rauschdichte des Verstärkers wurde für verschiedene Vorströme und Bandbreiteneinstellspannungen gemessen.
Die grafische Darstellung in Figur 13 zeigt die gemessenen Kurven im Vergleich zu den schematischen und analogen extrahierten Simulationen. Figur 14 zeigt das gesamte, integrierte Eingangsrauschen für unterschiedliche Verstärkerkonfigurationen. Die Kurve zeigt ein minimales Rauschen von *(sim. value) 0,6 $V_{RMS}$ für $I_{ss}$ = 250 $\mu$A.

**In-vivo-Aufnahme**

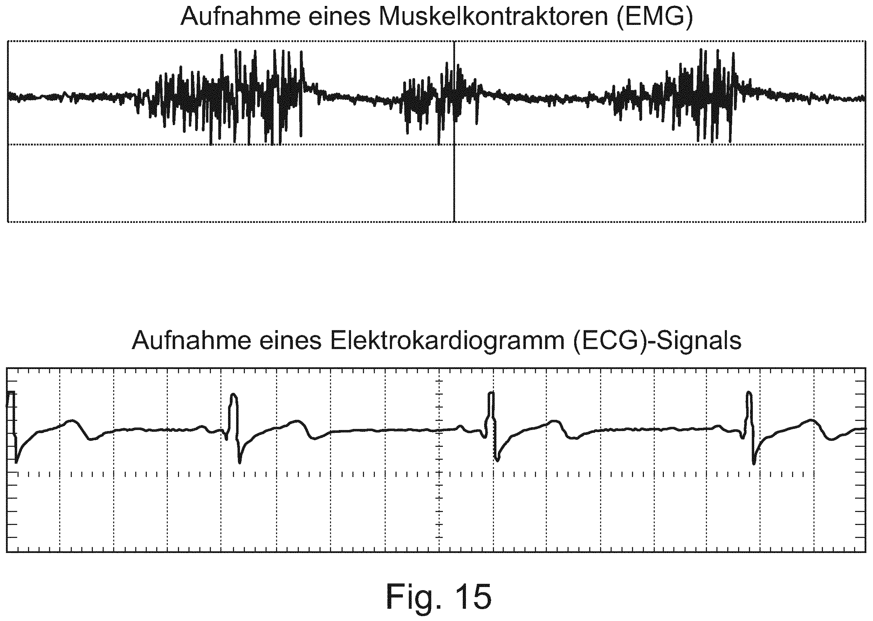

Das Erfassungssystem wurde mit bioelektrischen In-vivo-Signalen getestet, wie in Figur 15 gezeigt. Die bioelektrischen Signale wurden direkt aus den seriellen, digitalen Ausgängen durch Verwendung von SPI-Decodierung extrahiert. Der SPI-Bus (Serial Peripheral Interface) ist eine synchrone, serielle Kommunikationsinterfacespezifikation, die für Kommunikation auf kurze Entfernung verwendet wird. SPI-Geräte können durch Verwendung einer Master-Slave-Architektur mit einem einzigen Master in vollem Duplexbetrieb kommunizieren. Das Master-Gerät erzeugt den Frame für das Lesen und Schreiben. Eine Mehrzahl an Slave-Geräten wird durch Auswahl mit individuellen Slave-Select-Leitungen (SS) unterstützt.
Die obere Figur 15 zeigt drei aufeinanderfolgende Kontraktionen einer beispielhaften Bizeps-EMG-Erfassung. Die untere Figur 15 zeigt eine EKG-Erfassung.

**Schlussfolgerung**

Diese Arbeit zeigt die Umsetzung eines Biopotentialaufnahmesystems mit 8 Kanälen. In Tabelle 3 wird ein Vergleich mit ähnlichen Arbeiten gezeigt.
Auch wenn der beste Rausch-Effizienz-Faktor durch das Design erreicht wird, das BJT-Transistoren (BJT-Transistoren-->Mahdi-Referenz!) verwendet, hat dies den Nachteil, dass ein Restgleichstrom von 20 nA verbleibt, der auf Dauer zu Elektrodenkorrosion führen kann.
Der Kapazitätsmultiplizierer erfüllte seine Funktion der Bereitstellung eines breiten Bereichs für die obere Grenzfrequenz.
Da er jedoch für minimale Fläche und Leistungsverbrauch dimensioniert wurde, konnte das Rauschverhalten ohne Kapazitätsmultiplizierer nicht unterhalb von 1 $\mu V_{RMS}$ gehalten werden; das Rauschverhalten kann durch Software-Filtering aufrechterhalten werden.

Tabelle 3 zeigt einen Vergleich des gezeigten rauscharmen Verstärkers (LNA) mit anderen Systemen

| System | Verstärkung /db | Rauschen / µV$_{RMS}$ | Leistung/ Kanal /µW | fcL /Hz | fcU /Hz | CMR R /db | NRF |
|---|---|---|---|---|---|---|---|
| [6] | 40; 77 | 4,9 | 49 | 0,01- 1 k | 700-10 k | 139 | 7,84 |
| [7] | 80 | 0,291 | 2400 (5 V) | DC | 5 k | 82 | 3,57 |
| [8] | 40 | 2,2 | 12 (IV) | 0,3 | 10 k | 80 | 2,9 |
| [9] | >100 | 1,9(10 kHz) | 576 (1,8 V) | DC | 20 k | >99 | 12,9 |
| Vorher. [3] | 41-45 | 0,8-2,7 | 3,3-3300 | 0,2-10 k | 38-11k | 78 | 8,9-15 |
| Diese Arbeit | 39,3 oder 58,4 | 1,94 - 0,69* (sim val) | 303-1200* (sim) | 0.1 -10 k | 200 - 20 k | 60,3; 74 | 3.52 (1. Stufe) 6.57 (2. Stufe) |

Im Vergleich zur vorhergehenden Arbeit in [3] integrierte die vorliegende Ausgestaltung die übrigen Blöcke des angestrebten Systems.
Die Verstärkerfläche wurde um einen Faktor vier und einen analogen Ausgang reduziert.

**Ergebnis**

Die Erfindung zeigt die erfolgreiche Umsetzung und den Test eines vielseitig einsetzbaren bioelektrischen Signalerfassungschips mit 8 Kanälen. Die verstärkten Kanäle werden von zwei analogen Multiplexern ausgewählt und werden durch zwei SPI-kompatible 16-Bit-Datenströmen ausgegeben. Das gesamte integrierte Eingangsgeräusch kann für Bandbreiten zwischen 1 Hz und 10 kHz auf *(sim. value) 0,6 µV$_{RMS}$ herabjustiert werden. Das Erfassungssystem wurde für EKG- und EMG-Anwendungen getestet.

REFERENZEN

[1] B. Razavi, Design of analog CMOS integrated circuits: Tata McGraw-Hill Education, 2002.

[2] P. Harpe, H. Gao, R. van Dommele, E. Cantatore, and A. van Roermund, "21.2 A 3nW signalacquisition IC integrating an amplifier with 2.1 NEF and a 1.5 fJ/conv-step ADC," in Solid-State Circuits Conference-(ISSCC), 2015 IEEE International, 2015, S. 1-3.

[3] O. F. Cota, D. Plachta, T. Stieglitz, Y. Manch, and M. Kuhl, "In-vivo characterization of a 0.8-3 \muV RMS input-noise versatile CMOS pre-amplifier,"in Neural Engineering (NER), 2015 7th International IEEE/EMBS Conference on, 2015, S. 458-461.

[4] J. Ramirez-Angulo, S. R. Garimella, A. J. López-Martin, and R. G. Carvajal, "Gain programmable current mirrors based on current steering," Electronics Letters, vol. 42, no. 10, S. 559-560, 2006.

[5] J. A. Ruiz, A. Lopez-Martin, and J. Ramirez-Angulo, "Three novel improved CMOS capacitance scaling schemes," in Circuits and Systems (ISCAS), Proceedings of 2010 IEEE International Symposium on, 2010, S. 1304-1307.

[6] M. Yin and M. Ghovanloo, "A low-noise clockless simultaneous 32-channel wireless neural recording system with adjustable resolution," Analog Integrated Circuits and Signal Processing, vol. 66, no. 3, S. 417-431, ISI:000287319400010, 2011.

[7] J. Taylor and R. Rieger, "A low-noise front-end for multiplexed ENG recording using CMOS technology," Analog Integrated Circuits and Signal Processing, vol. 68, no. 2, S. 163-174, ISI:000292649900004, 2011.

[8] F. Zhang, J. Holleman, and B. P. Otis, "Design of ultra-low power biopotential amplifiers for biosignal acquisition applications," Ieee Transactions on Biomedical Circuits and Systems, vol. 6, no. 4, S. 344-355, 2012.

**Patentansprüche**

1.  Biokompatibles Aufnahmesystem zum Erfassen von elektronischen Informationen aus dem neuronalen System eines Lebewesens, umfassend:

    - einen Vorverstärker,
    - eine zweite Verstärkerstufe, wobei ein Eingang des Verstärkers der zweiten Stufe an einen Ausgang des Vorverstärkers gekoppelt ist,
    - einen Tiefpassfilter mit einem Kapazitätsmultiplizierer, der mit dem Verstärker der zweiten Stufe verbunden ist.

2.  Aufnahmesystem nach Anspruch 1 **dadurch gekennzeichnet, dass** der Vorverstärker P-MOS Eingangstransistoren in der ersten Verstärkerstufe verwendet.

3.  Aufnahmesystem nach Anspruch 1 **dadurch gekennzeichnet, dass** das Aufnahmesystem wenigstens zwei Signale unabhängig voneinander mit Hilfe von wenigstens zwei Aufnahmekanälen erfassen kann.

4.  Aufnahmesystem nach Anspruch 1, **dadurch gekennzeichnet, dass** das System durch Variation vorgegebener Signale variable untere (fcL) und obere (fcU) Eckfrequenzen aufweist.

5.  Aufnahmesystem nach Anspruch 2, **dadurch gekennzeichnet, dass** die erste Verstärkerstufe des Vorverstärkers aus einer vollständig differentiellen Teleskoparchitektur (Fully-differential Telesopic architecture) besteht.

6.  Aufnahmesystem nach Anspruch 1, wobei in dieses ein Flip-Flop-basierter Parallel-Seriell-Wandler integriert ist.

7.  Aufnahmesystem nach Anspruch 1, **dadurch gekennzeichnet, dass** der Tiefpassfilter ein variabler RC-Tiefpassfilter ist.

**Claims**

1.  A biocompatible recording system for acquiring electronic information from the neural system of a living being, comprising:

    - a preamplifier,
    - a second amplifier stage, wherein an input of the amplifier of the second stage is coupled to an output of the preamplifier,
    - a low-pass filter having a capacitance multiplier connected to the amplifier of the second stage.

2.  The recording system according to claim 1, **characterized in that** the preamplifier uses P-MOS input transistors in the first amplifier stage.

3.  The recording system according to claim 1, **characterized in that** the recording system can acquire at least two signals independently of one another with the aid of at least two recording channels.

4.  The recording system according to claim 1, **characterized in that** the system has variable lower (fcL) and upper (foil) corner frequencies by variation of predetermined signals.

5.  The recording system according to claim 2, **characterized in that** the first amplifier stage of the preamplifier consists of a fully-differential telescopic architecture.

6.  The recording system according to claim 1, in which a flip-flop-based parallel-serial converter is integrated.

7.  The recording system according to claim 1, **characterized in that** the low-pass filter is a variable RC low-pass filter.

**Revendications**

1.  Système d'enregistrement biocompatible pour détecter des informations électroniques à partir du système neuronal d'un être vivant, comprenant :

- un préamplificateur,
- un second niveau amplificateur, dans lequel une entrée de l'amplificateur du second niveau est couplée au niveau d'une sortie du préamplificateur,
- un filtre passe-bas avec un multiplicateur de capacité, qui est relié à l'amplificateur du second niveau.

**2.** Système d'enregistrement selon la revendication 1, **caractérisé en ce que** le préamplificateur utilise des transistors d'entrée MOS à canal P dans le premier niveau amplificateur.

**3.** Système d'enregistrement selon la revendication 1, **caractérisé en ce que** le système d'enregistrement peut détecter au moins deux signaux indépendamment l'un de l'autre à l'aide d'au moins deux canaux d'enregistrement.

**4.** Système d'enregistrement selon la revendication 1, **caractérisé en ce que** le système présente des fréquences de coupure inférieures (/cL) et supérieures (fcU) variables en faisant varier des signaux prédéfinis.

**5.** Système d'enregistrement selon la revendication 2, **caractérisé en ce que** le premier niveau amplificateur du préamplificateur se compose d'une architecture télescopique entièrement différentielle (Fully-differential Telescopic architecture).

**6.** Système d'enregistrement selon la revendication 1, dans lequel un convertisseur parallèle-série basé sur une bascule est intégré dans celui-ci.

**7.** Système d'enregistrement selon la revendication 1, **caractérisé en ce que** le filtre passe-bas est un filtre passe-bas RC variable.

Blockdiagramm des LNA8 Aufnahmesystems

# Fig. 1

EP 3 419 503 B1

b) Teleskopisch mit Rückkopplungsschaltkreis

a) FD-Teleskopisch PMOS Transistor Ebene

Fig. 2

Vergleich 2 uVRMS gegen 500 nVRMS

Leistungskurven für vorgegebenes
Rauschverhalten

Fig. 3

Zweite Stufe

Fig. 4

Zweite Stufe OTA

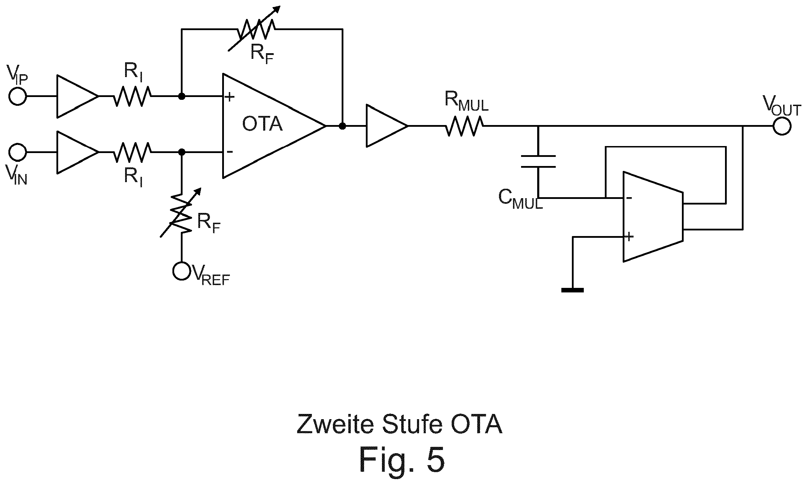

Fig. 5

Steuerstrom Kapazitätsmultiplizierer

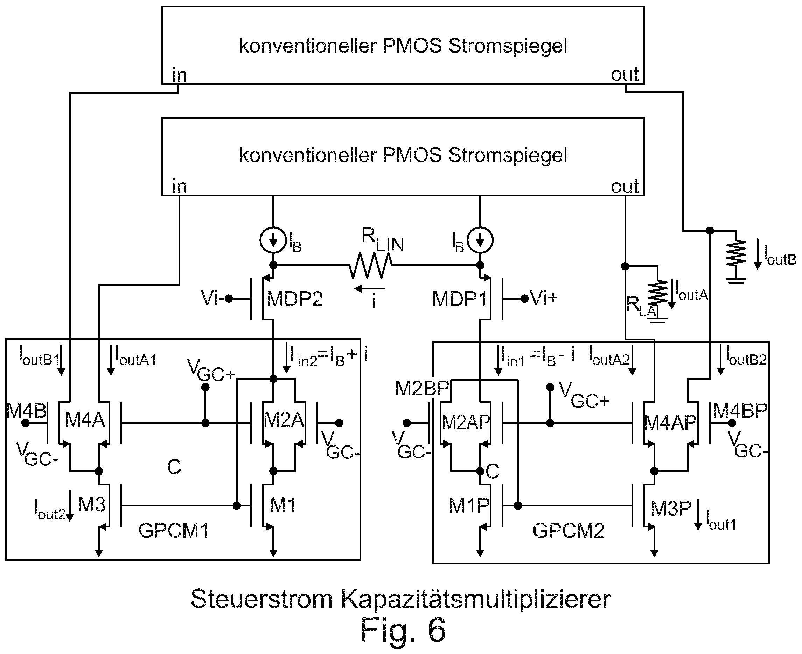

Fig. 6

Signalwege von Verstärkerausgängen zu seriellen Digitalausgängen

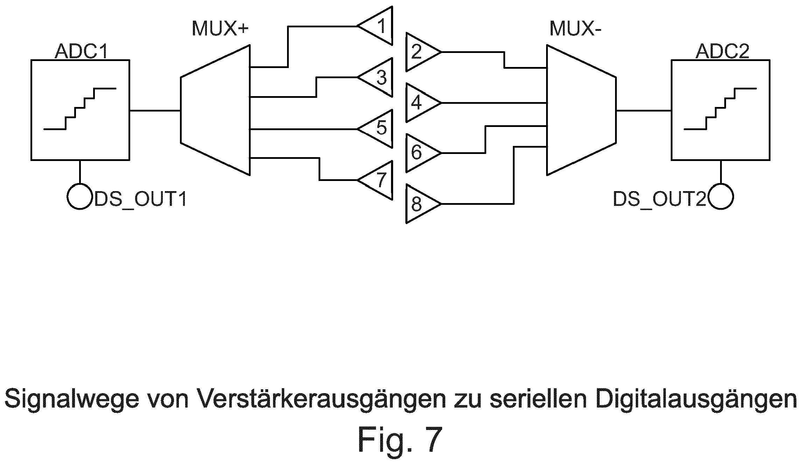

Fig. 7

EP 3 419 503 B1

Fig. 8

2 mm

Quelle  $C_{IN}$  Vorverstärker  Buff, CMFB  Kapazitätsmultiplizierer

2. Stufe

$V_{IN}$ CH5-8

CH5  CH6  CH7  CH8

$DS_{OUT2}$

ADC2

$DS_{OUT1}$

ADC1

$MUX_S$

CH1  CH2  CH3  CH4

$V_{IN}$ CH1-4

Mikroskopaufnahme des Chips mit 8 bipolaren Eingangskanälen

| Analog | | Digital | | Test (analog) | |
|---|---|---|---|---|---|
| (D, A) $V_{DD}$ | 3 | $I_{SS}$ (T1-T4) | 4 | $V_{EXT\_CMFB}$ | |
| (D, A) $V_{SS}$ | 3 | V_REF_ISS_EN | | $V_{DD\_ISS(1,5)}$ | 2 |
| $V_{REF\_ISS}$ | | LP_EN | | $V_{OUTN/P(1,5)}$ | 2 |
| $V_{IN(N/P)(1-8)}$ | 16 | RESET_EN | | $V_{OUT1/5}$ | 2 |
| $V_{REF\_CMFB}$ | | GAIN_0dB_EN | | Test (digital) | |
| $V_{GCP/N}$ | 2 | CAP_MULT_EN | | RESET ADC | |
| $V_{TUNE}$ | | CLK | | $Q1_{0-3}$ | 4 |
| $V_{REFH}$ | | $CHSEL_{0-3}$ | 4 | EOC1 | |
| $V_{REFL}$ | | DS OUT (1/2) | 2 | SW1 | |

I/O Pins des LNA8-Chips

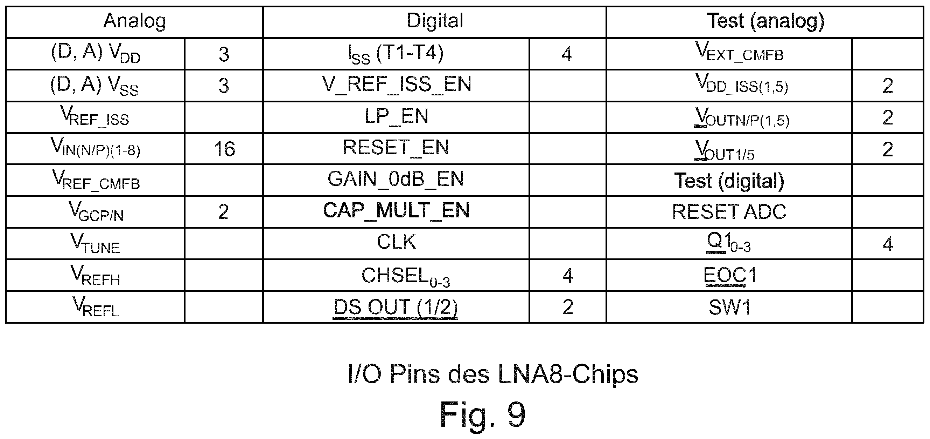

# Fig. 9

Variationen von unterer und oberer Grenzfrequenz über VTune und Kontrolle
der Vorspannung VGC+ des Kapazitätsmultiplizierers

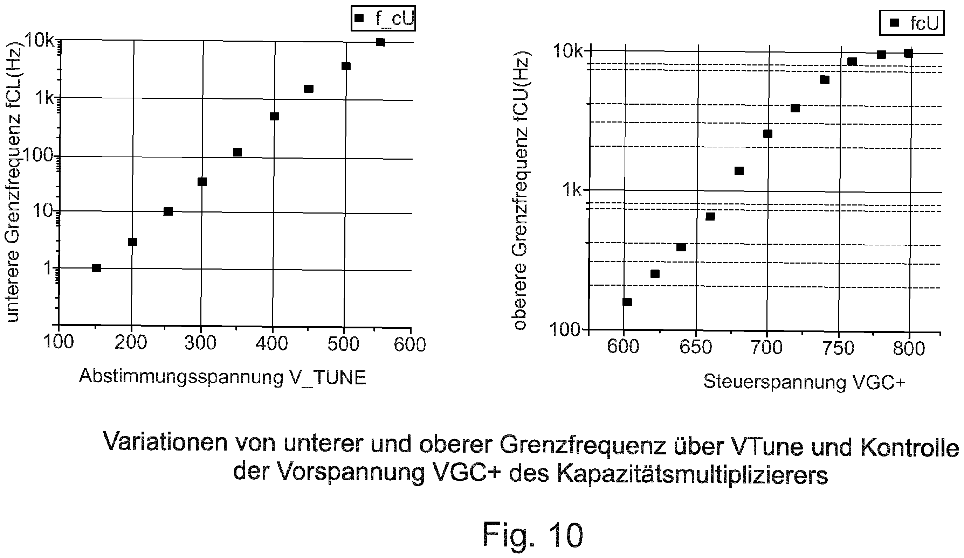

Fig. 10

EP 3 419 503 B1

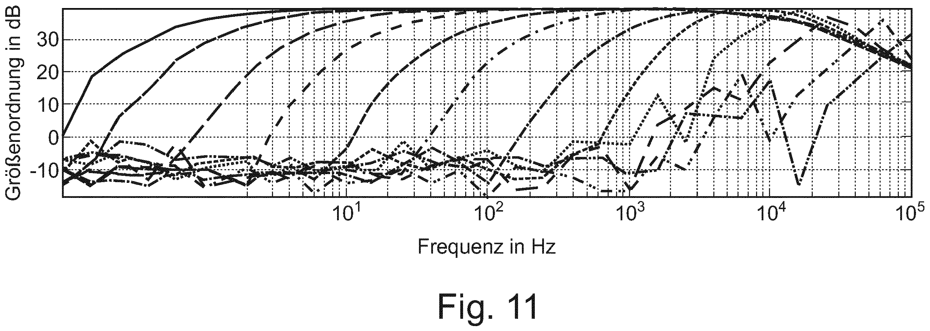

Fig. 11

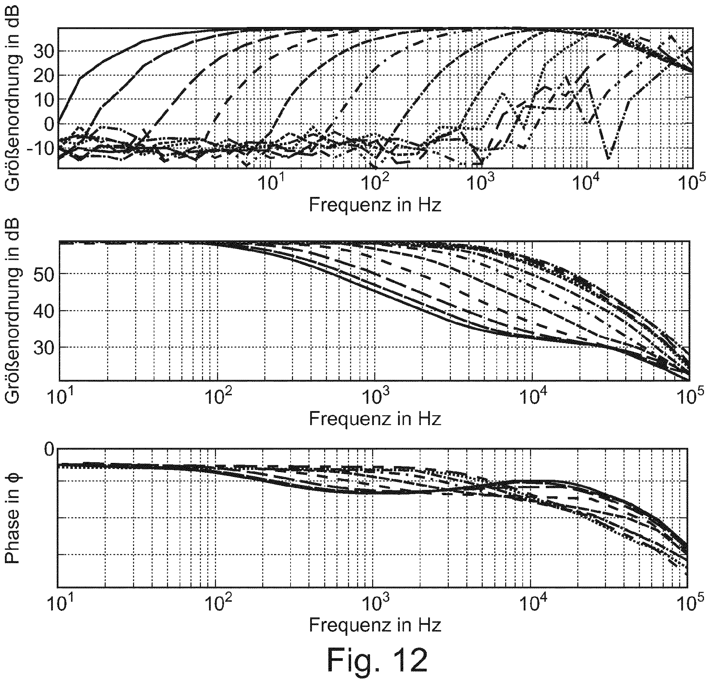

Fig. 12

Fig. 13

Ghovanloo-Typ erste Stufe

Eingangsbezogenes Rauschen (konstante Verstärkung angenommen)

Fig. 14

## Aufnahme eines Muskelkontraktoren (EMG)

## Aufnahme eines Elektrokardiogramm (ECG)-Signals

## Fig. 15

| System | Verstärkung /dB | Rauschen /$\mu V_{RMS}$ | Leistung / Kanal /$\mu W$ | fcL /Hz | fcU /Hz | CMRR /dB | NEF |
|---|---|---|---|---|---|---|---|
| [6] | 40, 77 | 4.9 | 4.9 | 0.01 - 1 k | 700 - 10 k | 139 | 7.84 |
| [7] | 80 | 0.291 | 2400 (5 V) | DC | 5 k | 82 | 3.57 |
| [8] | 40 | 2.2 | 12 (1 V) | 0.3 | 10 k | 80 | 2.9 |
| [9] | >100 | 1.9 (10 kHz) | 576 (1.8 V) | DC | 20 k | >99 | 12.9 |
| Prev. [3] | 41 - 45 | 0.8 - 2.7 | 3.3 - 3300 | 0.2 - 10 k | 38 - 11 k | 78 | 8.9 - 15 |
| This work | 40 - 60 ?? | XX - XX ? | XX - XX ? | 1 - 10 k ?? | 158 - 20 k?? | XX | XX ?? |

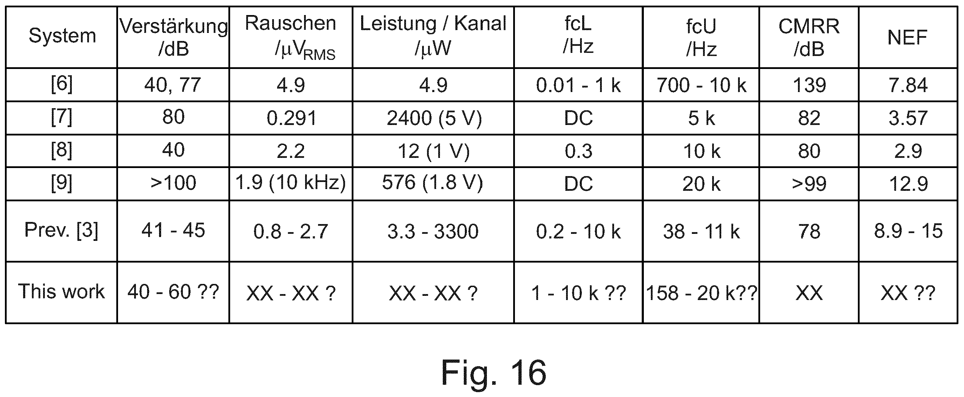

## Fig. 16

EP 3 419 503 B1

A. Basieren auf der Quelle
Nervenimpuls

Muskel Impuls

B. Basierend auf der Aufnahme
Aktionspotential

$V_m$

Aktionsstrom

$I_m$          $I_m$

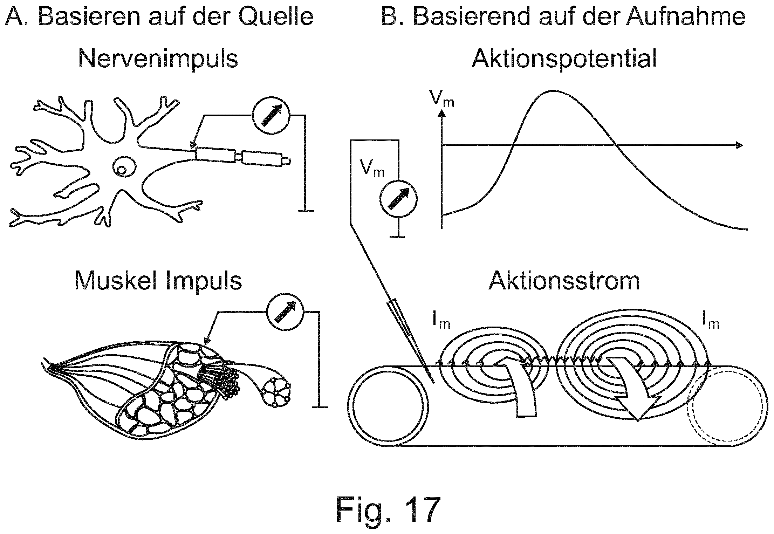

Fig. 17

EP 3 419 503 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2003097050 A1

**In der Beschreibung aufgeführte Nicht-Patentliteratur**

- **B. RAZAVI.** Design of analog CMOS integrated circuits. Tata McGraw-Hill Education, 2002
- **P. HARPE ; H. GAO ; R. VAN DOMMELE ; E. CANTATORE ; A. VAN ROERMUND.** 21.2 A 3nW signalacquisition IC integrating an amplifier with 2.1 NEF and a 1.5 fJ/conv-step ADC. *Solid-State Circuits Conference-(ISSCC), 2015 IEEE International,* 2015, 1-3
- **O. F. COTA ; D. PLACHTA ; T. STIEGLITZ ; Y. MANCH ; M. KUHL.** In-vivo characterization of a 0.8-3 \muV RMS input-noise versatile CMOS pre-amplifier. *Neural Engineering (NER), 2015 7th International IEEE/EMBS Conference on,* 2015, 458-461
- **J. RAMIREZ-ANGULO ; S. R. GARIMELLA ; A. J. LÓPEZ-MARTIN ; R. G. CARVAJAL.** Gain programmable current mirrors based on current steering. *Electronics Letters,* 2006, vol. 42 (10), 559-560
- **J. A. RUIZ ; A. LOPEZ-MARTIN ; J. RAMIREZ-ANGULO.** Three novel improved CMOS capacitance scaling schemes. *Circuits and Systems (ISCAS), Proceedings of 2010 IEEE International Symposium on,* 2010, 1304-1307
- **M. YIN ; M. GHOVANLOO.** A low-noise clockless simultaneous 32-channel wireless neural recording system with adjustable resolution. *Analog Integrated Circuits and Signal Processing,* 2011, vol. 66 (3), 417-431
- **J. TAYLOR ; R. RIEGER.** A low-noise front-end for multiplexed ENG recording using CMOS technology. *Analog Integrated Circuits and Signal Processing,* 2011, vol. 68 (2), 163-174
- **F. ZHANG ; J. HOLLEMAN ; B. P. OTIS.** Design of ultra-low power biopotential amplifiers for biosignal acquisition applications. *Ieee Transactions on Biomedical Circuits and Systems,* 2012, vol. 6 (4), 344-355